Europäisches Patentamt

European Patent Office

Office européen des brevets

(19)

(11) Veröffentlichungsnummer: **0 259 749 B1**

(12)    **EUROPÄISCHE PATENTSCHRIFT**

(45) Veröffentlichungstag der Patentschrift: **14.08.91**

(51) Int. Cl.5: **A61K 9/20**, A61K 9/70

(21) Anmeldenummer: **87112712.2**

(22) Anmeldetag: **01.09.87**

(54) Darreichungs- und Dosierungsform für Arzneimittelwirkstoffe, Reagentien oder dergleichen sowie Verfahren zu deren Herstellung.

(30) Priorität: **09.09.86 DE 3630603**

(43) Veröffentlichungstag der Anmeldung:
**16.03.88 Patentblatt 88/11**

(45) Bekanntmachung des Hinweises auf die
Patenterteilung:
**14.08.91 Patentblatt 91/33**

(84) Benannte Vertragsstaaten:
**AT BE CH DE ES FR GB GR IT LI LU NL SE**

(56) Entgegenhaltungen:
EP-A- 0 019 929        EP-A- 0 219 762
DE-A- 1 947 684        DE-A- 2 746 414
FR-A- 1 382 158        GB-A- 2 022 999

(73) Patentinhaber: **Desitin Arzneimittel GmbH
Weg beim Jäger 214
W-2000 Hamburg 63(DE)**

(72) Erfinder: **Schmidt, Wolfgang, Dr.
Reembroden 44
W-2000 Hamburg 63(DE)**

(74) Vertreter: **UEXKÜLL & STOLBERG Patentanwälte
Beselerstrasse 4
W-2000 Hamburg 52(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

EP 0 259 749 B1

## Beschreibung

Arzneimittel können in Form von Pulvern, Tropflösungen oder Säften oral verabreicht werden. Da bei diesen Abgabeformen eine genaue Dosierung jedoch schwierig ist, werden vom Hersteller dosierte Applikationsformen wie Tabletten, Dragees oder Kapseln generell bevorzugt. Auch Reagentien und andere Wirkstoffe, z.B. Süßstoffe, werden für eine genaue dosierte Anwendung häufig tablettiert. Die Herstellungstechnik für Tabletten, Dragees, Kapseln und dergleichen ist zwar weitgehend ausgereift, doch sind eine Reihe von systembedingten Nachteilen nicht zu übersehen.

Für niedrig dosierte Wirkstoffe muß ein großer Anteil an Hilfsstoffen zugesetzt werden, um zu einer handhabbaren Größe der Einzeldosis zu gelangen. Weiterhin ist eine genaue Kennzeichnung einzelner Tabletten oder Dragees praktisch nicht möglich. Es haben sich deshalb Durchdrückpackungen durchgesetzt, welche eine Mehrzahl von Tabletten, Dragees oder auch Kapseln enthalten und welche mit den notwendigen Informationen, insbesondere dem Namen des Präparates bedruckt sind. Die Herstellung solcher Verpackungen erfordert naturgemäß einen zusätzlichen Arbeitsgang und es werden Umverpackungen in Form von Faltschachteln benötigt, welche ein beträchtliches Leervolumen aufweisen und dadurch zusätzlich Lagerraum beanspruchen. Ein besonders gravierender Nachteil von Dragees und Kapseln besteht darin, daß eine Zerteilung praktisch unmöglich ist, die kleinste Dosis somit vorgegeben ist. Auch bei Tabletten ist eine genaue Zerteilung schwierig, lediglich größere Tabletten mit einer Kerbe als Sollbruchstelle lassen sich allenfalls teilen, wobei häufig ungleichgroße Bruchstücke entstehen.

Es sind bereits Versuche zur Schaffung einer neuen Darreichungsform für die orale Verabreichung von Arzneimitteln bekannt geworden, welche aus wirkstoffhaltigen Folien bestehen. Gemäß der BE-PS 637 363 wird ein papierartiges Trägermaterial aus unlöslichen Zellulosefasern mit einer Wirkstofflösung getränkt bzw. beschichtet und eine Dosierung durch Perforation der Trägerfolie nach Art eines Briefmarkenbogens erreicht. Aus den DE-OS 24 32 925 und 24 49 865 ist es bekannt, Arzneimittelwirkstoffe in Folienbildner einzuarbeiten, bei denen es sich vorzugsweise um wasserlösliche Verbindungen wie Methyl- und Ethylzellulose, insbesondere aber Hydroxypropylzellulose, Hydroxyethylzellulose oder Methylhydroxypropylzellulose handelt. Auch die so erhaltenen wirkstoffhaltigen Folien lassen sich zur Dosierung durch Perforation in einzelne Abschnitte aufteilen. Aus DE-A-2746414 ist es ferner bekannt, derartige Dosierfolien mit weiterer Wirkstoffhaltigen oder- freien folien zu Dosierlaminaten zu vereinigen. Dadurch lassen sich inkompatible Wirkstoffe verarbeiten oder die Lösungsgeschwindigkeit bereinflussen. Diese Laminate insgesamt werden in form von Dosiereinheiten verwendet. Diese Vorschläge haben keinen Eingang in die Praxis gefunden und in dem neuesten Lehrbuch der "Arzneiformenlehre" von P.H. List, 4. Auflage, Stuttgart, 1985, finden sie keine Erwähnung. Dies beruht ersichtlich darauf, daß die bislang bekanntgewordenen Vorschläge es nicht ermöglichen, die geforderte Gewichtskonstanz und gleichmäßige Wirkstoffverteilung zu erreichen, welche heute gefordert werden. Die Ph. Eur. setzt zum Beispiel Maßstäbe für die Gleichförmigkeit des Gewichtes einzeldosierter Arzneiformen, wobei diese dem jeweiligen Durchschnittsgewicht entsprechend nach höchstzulässigen Abweichungen in % gestaffelt sind. Die Forderung liegt im allgemeinen bei +/- 5 bis max. 10%. Entsprechende Werte für feste Arzneiformen bestehen auch hinsichtlich anderer Parameter wie Zerfallzeit und Lösungsgeschwindigkeit.

Die oben erwähnten Vorschläge des Standes der Technik führen zu Produkten ungenügender Akzeptanz durch die Patienten (Papierabschnitte lassen sich nur schlecht einnehmen) und erlauben keine exakte Dosierung pro Flächeneinheit, wie sie unbedingt gefordert werden muß. Bei Inkorporieren des Wirkstoffes in eine Folie bereitet nicht nur die genaue Dosierung Schwierigkeiten, sondern ein wesentlicher weiterer Nachteil besteht darin, daß für jeden Wirkstoff eine entsprechende Folie gesondert hergestellt werden muß, so daß die Wirtschaftlichkeit des Herstellungsverfahrens nicht gegeben ist.

Der Erfindung liegt demgegenüber die Aufgabe zugrunde, eine "zweidimensionale" Darreichungs- und Dosierungsform zu schaffen, welche die genannten Nachteile nicht aufweist, sich leicht herstellen läßt und mit großer Flexibilität an die Anforderungen des Marktes und verschiedener Wirkstoffe angepaßt werden kann.

Gegenstand der Erfindung ist eine Darreichungs- und Dosierungsform für Arzneimittelwirkstoffe, Reagentien oder dergleichen in Form eines folienförmigen Trägermaterials mit einer wirkstoffhaltigen Beschichtung, wobei diese Darreichungsform dadurch gekennzeichnet ist, daß das Trägermaterial ein Release-Papier, ein Release-Film oder eine Release-Folie ist und daß das Trägermaterial einseitig mit der wirkstoffhaltigen Beschichtung versehen ist, welche nach Vorzerteilung in Dosiseinheiten von dem Trägermaterial dosisweise abziehbar ist.

Die erfindungsgemäße Darreichungsform weist mehrere wesentliche Vorteile auf:
- Da der Träger im Gegensatz zu den vorbekannten Ausführungsformen keinen Teil der Darreichungsform bildet, kann er die erforderliche Festigkeit aufweisen, ohne die Akzeptanz des Arzneimittels

2

durch Patienten zu beeinträchtigen,

- die wirkstoffhaltige Schicht kann bei hochwirksamen Arzneimitteln sehr dünn sein, da das Trägermaterial die mechanische Festigkeit gewährleistet,
- mit Hilfe moderner Auftragverfahren läßt sich die wirkstoffhaltige Beschichtung mit konstanter Schichtdicke aufbringen, so daß die erforderlichen Toleranzen eingehalten werden können,
- falls eine Sterilisierung erforderlich ist, kann diese wegen der geringen Schichtdicke problemlos mittels Strahlenbehandlung erreicht werden,
- der Träger läßt sich auf der Vorder- und insbesondere der Rückseite mit verschiedenen Informationen bedrucken,
- aufgrund der relativ großen Fläche von beispielsweise 4 bis 10 cm² lassen sich ausführliche Informationen für den Benutzer auf das unbeschichtete Trägermaterial oder auch nachträglich aufdrucken,
- die Dosiseinheiten lassen sich durch entsprechende Vorzerteilung flexibel gestalten, so daß für verschiedene Dosierungen (z.B. für Erwachsene und Kinder) nur ein Produkt hergestellt werden muß; die Vorzerteilung kann ggf. auch erst in der Apotheke oder im Krankenhaus nach ärztlichen Angaben vorgenommen werden.

Mit den vorbekannten Darreichungsformen in Folienform hat die erfindungsgemäße Darreichungsform darüberhinaus den Vorteil des äußerst geringen Platzbedarfes gemeinsam. Statt Faltschachteln können daher beispielsweise Taschen oder Beutel aus Kunststoffolie oder kunststoffbeschichtetem Papier verwendet werden, in welche das Produkt eingesiegelt wird, ähnlich wie feuchte Erfrischungstücher.

Als Trägermaterialien eignen sich die verschiedensten Materialien, beispielsweise Papiere mit einem Gewicht von etwa 80 bis 120, vorzugsweise 100 g/m², Kunststoffilme bzw. -folien auf Basis von Polyethylen, Polyvinylchlorid, Polyvinylidenchlorid, Polyester und anderen indifferenten Polymeren oder dünne Metallfolien, beispielsweise solche aus Aluminium. Bevorzugt werden siliconisierte Papiere, welche in unterschiedlichen Qualitäten im Handel erhältlich sind, und welche insbesondere zur Abdeckung von selbstklebenden Produkten wie Pflastern, Klebebändern oder Haftetiketten Verwendung finden. Die an sich auch geeigneten mit Wachs oder Paraffin beschichten Release-Papiere sind dagegen in der Praxis weitgehend durch die mit inerten Siliconen beschichteten Papiere ersetzt worden. Bei einem Auftrag der wirkstoffhaltigen Beschichtung auf nur eine Seite der Trägerfolie reicht es aus, wenn nur diese mit einer nicht haftenden Beschichtung versehen ist. Die Rückseite sollte dagegen vorzugsweise so beschaffen sein, daß sie mit Informationen unterschiedlicher Art gut und dauerhaft bedruckbar ist.

Die Möglichkeit der vorder- und rückseitigen Bedruckung ist ein besonderer Vorteil der erfindungsgemäßen Darreichungsform. Beispielsweise können die Kennzeichnung, Angaben über die Inhaltsstoffe sowie Dosierungsangaben aufgedruckt werden. Gegebenenfalls läßt sich sogar der ganze Inhalt eines Beipackzettels rückseitig aufdrucken mit der Folge, daß ein separater Beipackzettel, der auch häufig verlorengeht, überflüssig wird. Bei Arzneimitteln, welche regelmäßig genommen werden müssen, beispielsweise bei hormonalen Contrazeptiva, kann der gesamte Verabreichungsplan so angebracht werden, daß eine einfache Einnahmekontrolle gewährleistet ist. Da die einzelnen Dosiseinheiten von dem Träger abgezogen werden, bleibt dieser bis zum vollständigen Aufbrauch des Arzneimittels erhalten und es gehen keine der aufgedruckten Informationen verloren.

Für die wirkstoffhaltige Beschichtung findet vorzugsweise eine wässrige Beschichtungsmasse Verwendung, die physiologisch inert ist und deren Einzelkomponenten für Arzneimittel bzw. Lebensmittel geeignet sind. Dabei handelt es sich zum einen um wasserlösliche Quellstoffe in der Art polymerer Filmbildner, vorzugsweise Gelatine, Zellulosen oder Hemizellulosen, quellende oder lösliche Stärken. Vorzugsweise werden ferner Weichmacher zugesetzt, insbesondere mehrwertige Alkohole wie Glycerin oder Sorbitol. Zur Einstellung der erwünschten Viskosität der Beschichtungsmasse, welche etwa die Konsistenz eines Schleimes aufweist, finden polymere Quellstoffe Verwendung, vorzugsweise Alginate, Pectine, Chitine, Lecithine oder Polyethylenglykole. Diese letzteren Stoffe können gleichzeitig als Haftvermittler dienen. Andererseits können auch wasserlösliche Gumme oder Gummi arabicum zugesetzt werden, um die Haftung der Beschichtung auf dem Trägermaterial zu verbessern. Schließlich können noch Konservierungsmittel wie z.B. p-Hydroxybenzoesäureester, Farbstoffe (Lebensmittelfarbstoffe), Pigmente wie Titandioxid oder Aroma- und Süßstoffe zugesetzt werden.

Coatingmassen mit einem Wassergehalt von ungefähr 50% und einer Viskosität von etwa 30 bis zu 10000 cPs haben sich als besonders geeignet erwiesen. Die Rezeptur und Herstellung ähnelt derjenigen eines Arzneimittelsaftes, in welchem der Wirkstoff bzw. die Wirkstoffkombination gelöst oder gleichmäßig dispergiert wird. Die Beschichtungsmasse muß ausreichende Homogenität und galenische Stabilität aufweisen, damit ein gleichmäßiger Wirkstoffgehalt der fertigen Beschichtung sichergestellt ist.

Folgende Rahmenrezeptur hat sich bewährt:

3

| Gelatine | 8 bis 10 g |
| Stärke | 3 bis 8 g |
| Glycerin | 1 bis 2 g |
| Wasser | 30 bis 50 g |

In dieser Grundmasse wird der Wirkstoff gelöst bzw. dispergiert. Im Fall der Verwendung einer Dispersion muß der Wirkstoff für eine gleichmäßige Verteilung äußerst feinteilig sein. Vorzugsweise liegt die mittlere Teilchengröße im Bereich von etwa 1 bis 20 μm.

Die gewünschte Dosis des Wirkstoffes und die angestrebte Fläche der Dosiseinheiten bestimmen letztlich die Dicke der Schicht, wobei der Feuchtigkeitsgehalt der Beschichtungsmasse und der fertigen Beschichtung zu berücksichtigen sind.

Im Rahmen der Erfindung ist es auch möglich, die Beshichtungsmasse zu einer wirkstoffhaltigen Folie zu verarbeiten und diese anschließend, gegebenenfalls unter Verwendung eines physiologisch einsetzbaren inerten Klebstoffes, auf das Trägermaterial aufzukaschieren. Diese Ausführungsform kommt insbesondere dann in Betracht, wenn die wirkstoffhaltige Beschichtung eine größere Dicke aufweisen soll, so daß die Verarbeitung zu einer Folie möglich und sinnvoll ist.

Die erfindungsgemäße Darreichungsform ist besonders geeignet für Arzneimittel, welche niedrig dosiert verabreicht werden, d.h. bei welchen die Einzeldosis für die orale Applikation zwischen 0 mg (Placebo) und etwa 20 mg liegt. Geeignete Arzneimittelwirkstoffe finden sich in allen Bereichen der oralen Therapie; hervorzuheben sind u.a. Analeptika, Antibiotika, Antidiabetika, Antiemetika, Antiepileptika, Antihypertonika, Cortikoide, Geriatrika, Hypnotika, Cardiaka, Hypostatika und Biowirkstoffe.

Die Beschichtung kann einen oder mehrere Arzneimittelwirkstoffe enthalten. Falls bei Verwendung mehrerer Wirkstoffe diese nicht ohne weiteres miteinander verträglich sind, ist es bei der erfindungsgemäßen Darreichungsform möglich, die Beschichtung in mehreren Schichten unterschiedlicher Zusammensetzung aufzubringen und die Wirkstoffe dadurch voneinander zu trennen, wobei erforderlichenfalls eine wirkstofffreie Zwischenschicht vorgesehen werden kann. Weiterhin ist es möglich, über der wirkstoffhaltigen Schicht noch eine weitere Schutzschicht vorzusehen, welche den/die Wirkstoff(e) gegen eine Berührung mit der Atmosphäre und/oder gegen Licht schützt. In diesen Fällen muß die Schutzschicht demgemäß luft- und feuchtigkeitsundurchlässig und/oder durch Zusatz entsprechender Farbstoffe bzw. Pigmente lichtundurchlässig sein.

Weiterhin kann durch entsprechenden Aufbau der Beschichtung die Wirkstoffabgabe nach Verabreichung des Arzneimittels gesteuert werden. Beispielsweise ist es möglich, eine Wirkstoffschicht zwischen mindestens zwei weiteren Schichten anzuordnen, welche die Wirkstoffresorption im Magen/Darmtrakt in an sich bekannter Weise steuern. Dabei kann die Wirkstoffschicht z.B. zwischen zwei säureunlöslichen Schichten angeordnet werden, so daß bei Verabreichung der Magen passiert wird und die Resorption erst im Darmtrakt erfolgt. In ähnlicher Weise können unterschiedliche Wirkstoffe in verschiedenen Schichten übereinander auf die Trägerfolie aufgebracht werden, damit die Resorption nacheinander und/oder verzögert erfolgt. Ähnliche pharmakokinetische Effekte lassen sich durch das Einarbeiten (z.B. Suspendieren) von unterschiedlich vorbehandelten mikroverkapselten Wirkstoffen erzielen.

Die Aufbringung der wirkstoffhaltigen Beschichtungen auf den Träger, z.B. ein Release-Papier oder eine Release-Kunststoffolie, erfolgt vorzugsweise mit Hilfe eines Glattwalzen-Beschichtungsverfahrens. Die vorzugsweise auf ca. 60 bis 80°C erwärmte Beschichtunsmasse wird dabei au einem geschlossenen Auftragssystem auf eine beheizte Walze in dünner Schicht übertragen. Mit verzögertem Gleichlauf in bestimmten wählbaren Verhältnissen wird die Masse auf eine parallel angeordnete Walze übertragen, wobei eine Reduzierung der Schichtdicke im Verhältnis 1:2 bis 1:10 erfolgen kann, wodurch gleichzeitig die Toleranzen bei der Auftragung um diese Faktoren verringert werden. Im Gleichlauf erfolgt dann über ein weiteres Walzensystem die Beschichtung des Trägermaterials. Bei einer Anpassung der Wirkstoffbeschichtungsmasse an den Release-Wert des Trägermaterials kann auf den Zusatz eines Klebemittels völlig verzichtet werden. Gegebenenfalls können jedoch auch geeignete Haftvermittler zugesetzt werden.

Bei Aufbringung mehrerer Schichten, wie dies oben bereits beschrieben wurde, werden diese nacheinander aufgebracht, wobei ggf. jede Beschichtung zuvor eine Trocknungsstation durchläuft. Diese kann beispielsweise aus einem temperierten Walzenpaar und einem in Sektionen steuerbaren Trockentunnel bestehen. Nach dem letzten Beschichtungsvorgang wird das beschichte Material auf Rollen aufgewickelt.

Die wirkstoffhaltige Beschichtung wird anschließend in Dosiseinheiten vorzerteilt, welche ähnlich wie Haftetiketten vom Trägermaterial abziehbar sind. Normalerweise wird diese Vorzerteilung beim Arzneimittelhersteller erfolgen, es ist jedoch auch denkbar, das beschichtete Material beispielsweise an Krankenhäuser oder Apotheken auszuliefern, wo dann die Vorzerteilung dosisabhängig oder auch individuell nach ärztlicher Vorgabe durchgeführt werden kann.

Die Vorzerteilung erfolgt in besonders einfacher Weise durch Stanzung, wobei es möglich ist, diesen Schritt mit der Bedruckung des Trägermaterials zu kombinieren. In vielen Fällen wird es allerdings günstiger sein, die Bedruckung des Trägermaterials vor der Beschichtung vorzunehmen.

Vor oder besser nach Vorzerteilung der wirkstoffhaltigen Beschichtung in Dosiseinheiten wird das beschichtete Trägermaterial zu gebrauchsfertigen Abschnitten zerschnitten, welche eine bestimmte Anzahl von Dosiseinheiten enthalten. Es ist auch denkbar, das Material auf Rollen zu schmalen Bändern zu zerschneiden. Von einer solchen Einzelrolle können dann die einzelnen Dosiseinheiten ähnlich wie einzelne Haftetiketten abgezogen werden.

Vorstehend wurde die Erfindung im wesentlichen im Zusammenhang mit Arzneimitteln beschrieben, worauf sie jedoch keineswegs beschränkt ist. Beispielsweise lassen sich in derselben Weise auch Dosierungsformen für chemische Reagentien, Aromastoffe und dergleichen herstellen.

Zur näheren Erläuterung der Erfindung sollen die nachfolgenden Ausführungsbeispiele dienen.

Beispiel 1
Herstellung eines Cardiakum

Zum Naßauftrag auf ein Releasepapier (Silikonpapier mit einem Flächengewicht von 100 g/m$^2$) wurde eine Beschichtungsmasse gemäß folgender Rezeptur hergestellt:

| Gelatine | 10,0 | Gew.-Teile | = | 22,22% |
|---|---|---|---|---|
| Kartoffelstärke | 3,0 | -"- -"- | = | 6,67% |
| Glycerin | 1,5 | -"- -"- | = | 3,33% |
| Titandioxid | 0,3 | -"- -"- | = | 0,67% |
| α-Acetyldigoxin | 0,2 | -"- -"- | = | 0,44% |
| Wasser | 30,0 | -"- -"- | = | 66,67% |

Diese Beschichtungsmasse wurde in einer Schichtdicke von 90 g/m$^2$ mittels Walzen auf das Releasepapier aufgebracht. Nach dem Trocknen wies die Beschichtung einen Restwassergehalt von 11,76% auf. Das Beschichtungsgewicht lag bei 34 g/m$^2$, was einem Arzneimittelanteil von 0,4 g/m$^2$ entspricht. Ein Abschnitt von 2 × 2,5 cm = 5 cm$^2$ (entsprechend den Abmessungen einer üblichen Briefmarke) enthält 0,2 mg α-Acetyldigoxin, was mit dem Gehalt der handelsüblichen Tabletten übereinstimmt.

Beispiel 2
Herstellung eines Contrazeptivum

Zum Naßauftrag auf ein Releasepapier (einseitig siliconisiertes Papier von 110 g/m$^2$) wurde eine Beschichtungsmasse von schleimartiger Konsistenz nach folgender Rezeptur hergestellt:

| Gelatine | 10,00 | Gew.-Teile | = | 22,222% |
|---|---|---|---|---|
| Maisstärke | 3,17 | -"- -"- | = | 7,044% |
| Glycerin | 1,50 | -"- -"- | = | 3,333% |
| Titandioxid | 0,30 | -"- -"- | = | 0,667% |
| Levonorgestrel | 0,03 | -"- -"- | = | 0,067% |
| Wasser | 30,00 | -"- -"- | = | 66,663% |

Die Beschichtungsmasse wurde mittels eines Walzenübertragungsverfahrens mit einem Beschichtungsgewicht von 45 g/m$^2$ auf das Releasepapier aufgebracht. Nach dem Trocknen wies die Beschichtung einen Restwassergehalt von 11,76% auf. Bei einem Beschichtungsgewicht von 17 g/m$^2$ betrug der Arzneimittelanteil 0,03 g/m$^2$.

Ein Abschnitt von 2,5 × 4 cm bzw. zwei Abschnitte von 2,5 × 2 cm = 10 cm$^2$ enthalten somit 0,03 mg Levonorgestrel, was dem Gehalt der handelsüblichen Dragees entspricht.

## Patentansprüche

1. Darreichungs- und Dosierungsform für Arzneimittelwirkstoffe, Reagentien, Aromastoffe oder dergleichen in Form eines folienförmigen Trägermaterials mit einer wirkstoffhaltigen Beschichtung, dadurch gekennzeichnet, daß das Trägermaterial ein Releasepapier, ein Releasefilm oder eine Releasefolie ist und daß das Trägermaterial einseitig mit der wirkstoffhaltigen Beschichtung versehen ist, welche nach Vorzerteilung in Dosiseinheiten von dem Trägermaterial dosisweise abziehbar ist.

2. Darreichungsform nach Anspruch 1, dadurch gekennzeichnet, daß das Trägermaterial ein silicon- oder wachsbeschichtetes Releasepapier ist.

3. Darreichungsform nach Anspruch 1 oder 2, dadurch gekennzeichnet, daß die wirkstoffhaltige Beschichtung durch Stanzung in Dosiseinheiten vorzerteilt ist.

4. Darreichungsform nach einem der Ansprüche 1 bis 3, dadurch gekennzeichnet, daß die Beschichtung einen oder mehrere Arzneimittelwirkstoffe enthält.

5. Darreichungsform nach einem der Ansprüche 1 bis 4, dadurch gekennzeichnet, daß die Beschichtung wasserlösliche Quellstoffe als polymere Filmbildner und gegebenenfalls Weichmacher enthält.

6. Darreichungsform nach einem der Ansprüche 1 bis 5, dadurch gekennzeichnet, daß sie zur Viskositätseinstellung polymere Quellstoffe enthält, welche gleichzeitig als Haftvermittler dienen können.

7. Darreichungsform nach einem der Ansprüche 1 bis 6, dadurch gekennzeichnet, daß die Beschichtung in mehreren Schichten unterschiedlicher Zusammensetzung aufgebracht ist.

8. Darreichungsform nach Anspruch 7, dadurch gekennzeichnet, daß miteinander inkompatible Wirkstoffe in getrennten Schichten nacheinander auf das Trägermaterial aufgebracht sind.

9. Darreichungsform nach Anspruch 7, dadurch gekennzeichnet, daß eine Wirkstoffschicht zwischen mindestens zwei weiteren Schichten angeordnet ist, welche die Wirkstoff-Resorption im Magen/Darmtrakt in an sich bekannter Weise steuern.

10. Darreichungsform nach Anspruch 7, dadurch gekennzeichnet, daß über der Wirkstoffschicht eine weitere Schicht aufgebracht ist, die den Wirkstoff gegen Berührung mit der Atmosphäre und/oder gegen Licht schützt.

11. Darreichungsform nach einem der Ansprüche 1 bis 10, dadurch gekennzeichnet, daß die Rückseite des Trägermaterials mit die Wirkstoffzusammensetzung und/oder deren Einnahme betreffenden Informationen bedruckbar ist.

12. Verfahren zur Herstellung der Arzneimitteldarreichungsform der Ansprüche 1 bis 11, dadurch gekennzeichnet, daß man eine wirkstoffhaltige Zusammensetzung mit Hilfe von Walzen auf die nichthaftend ausgerüstete Seite eines Releasepapiers, eines Releasefilms oder einer Releasefolie aufbringt.

## Claims

1. Presentation and dosage form for pharmaceutical active substances, reagents, aromas or the like in the form of a foil-like carrier material with an active-substance-containing coating, characterized in that the carrier material is a release paper, a release film or a release foil and that the carrier material is provided on one side with the active-substance-containing coating, which can be removed dosewise from the carrier material following prior division into dosage units.

2. Presentation form according to claim 1, characterized in that the carrier material is a silicone or wax-coated release paper.

3. Presentation form according to claims 1 or 2, characterized in that the active-substance-containing coating substance is pre-divided into dosage units by punching.

4. Presentation form according to one of claims 1 to 3, characterized in that the coating contains one or more pharmaceutical active substances.

5. Presentation form according to one of claims 1 to 4, characterized in that the coating contains water-soluble swelling substances as polymeric foil formers and optionally softeners.

6. Presentation form according to one of claims 1 to 5, characterized in that it contains, to set the viscosity, polymeric swelling substances, which can simultaneously serve as adhesion promoters.

7. Presentation form according to one of claims 1 to 6, characterized in that the coating is applied in the form of several layers having differing composition.

8. Presentation form according to claim 7, characterized in that incompatible active substances are applied one after the other as separate layers to the carrier material.

9. Presentation form according to claim 7, characterized in that an active substance layer is arranged between at least two other layers which control the absorption of the active substance in the gastro-intestinal tract in a manner known per se.

10. Presentation form according to claim 7, characterized in that a further layer is applied onto the active substance layer, said layer protecting the active substance against contact with the atmosphere and/or against light.

11. Presentation form according to one of claims 1 to 10, characterized in that the back of the carrier material can be printed with the active substance composition and/or information concerning the intake thereof.

12. Process for preparing the pharmaceutical presentation form according to claims 1 to 11, characterized in that an active-substance-containingcomposition is applied with the aid of rollers to the non-adhesively finished side of a release paper, a release film or a release foil.

**Revendications**

1. Forme de présentation ou de dosage de principes actifs médicamenteux, réactifs, substances aromatisantes ou similaires, sous la forme d'un matériau support en forme de feuille muni d'un revêtement contenant le principe actif, caractérisée en ce que le matériau support est un papier détachable, un film détachable ou une feuille détachable et, le matériau support est muni d'un côté du revêtement contenant le principe actif, que l'on peut détacher par doses du matériau support après l'avoir préalablement divisé en doses unitaires.

2. Forme de présentation selon la revendication 1, caractérisée en ce que le matériau support est un papier détachable revêtu de silicone ou de cire.

3. Forme de présentation selon la revendication 1 ou 2, caractérisée en ce que le revêtement contenant le principe actif est préalablement divisé en doses unitaires par poinçonnage.

4. Forme de présentation selon l'une quelconque des revendications 1 à 3, caractérisée en ce que le revêtement contient un ou plusieurs principe(s) actif(s) médicamenteux.

5. Forme de présentation selon l'une quelconque des revendications 1 à 4, caractérisée en ce que le revêtement contient des substances épaississantes, comme des agents filmogènes polymères et, le cas échéant, des plastifiants.

6. Forme de présentation selon l'une quelconque des revendications 1 à 5, caractérisée en ce qu'elle contient des substances épaississantes polymères pour ajustement de la viscosité, celles-ci pouvant servir en même temps d'agents adhésifs.

7. Forme de présentation selon l'une quelconque des revendications 1 à 6, caractérisée en ce que le

7

revêtement est constitué de plusieurs couches de compositions différentes.

8. Forme de présentation selon la revendication 7, caractérisée en ce que des principes actifs incompatibles entre eux sont appliqués successivement sur le matériau support, dans des couches séparées.

9. Forme de présentation selon la revendication 7, caractérisée en ce qu'une couche de principe actif est placée entre au moins deux autres couches qui règlent, par des moyens connus par eux-mêmes, la résorption du principe actif dans l'estomac/le tractus intestinal.

10. Forme de présentation selon la revendication 7, caractérisée en ce que l'on étale, sur la couche de principe actif, une couche supplémentaire qui préserve le principe actif, une couche supplémentaire qui préserve le lumière.

11. Forme de présentation selon l'une quelconque des revendications 1 à 10, caractérisée en ce que l'on peut imprimer au verso du matériau support la composition du principe actif et/ou des informations concernant sa prise.

12. Procédé pour préparer la forme de présentation de médicament des revendications 1 à 11, caractérisé en ce que l'on étale, à l'aide de cylindres, une composition contenant le principe actif sur le côté laissé non adhésif d'un papier détachable, d'un film détachable ou d'une feuille détachable.